(19) Europäisches Patentamt
European Patent Office
Office européen des brevets

(11) Veröffentlichungsnummer : **0 105 196**
**B1**

(12) # EUROPÄISCHE PATENTSCHRIFT

(45) Veröffentlichungstag der Patentschrift : **29.07.87**

(21) Anmeldenummer : **83108570.9**

(22) Anmeldetag : **31.08.83**

(51) Int. Cl.[4] : **C 07 C157/12**, C 07 C143/68, A 61 K 31/325// C07C143/833

(54) **Substituierte Benzolsulfonsäureester, Verfahren zu ihrer Herstellung und ihre Verwendung als Arzneimittel.**

(30) Priorität : **04.09.82 DE 3232959**

(43) Veröffentlichungstag der Anmeldung : **11.04.84 Patentblatt 84/15**

(45) Bekanntmachung des Hinweises auf die Patenterteilung : **29.07.87 Patentblatt 87/31**

(84) Benannte Vertragsstaaten : **AT BE CH DE FR GB IT LI LU NL SE**

(56) Entgegenhaltungen : **GB-A- 1 570 828**

(73) Patentinhaber : **HOECHST AKTIENGESELLSCHAFT**
**Postfach 80 03 20**
**D-6230 Frankfurt am Main 80 (DE)**

(72) Erfinder : **Rösner, Manfred, Dr.**
**Unter den Buchen 7**
**D-6239 Eppstein/Taunus (DE)**
Erfinder : **Urbanietz, Josef**
**Am Sandring 44**
**D-6231 Schwalbach (DE)**
Erfinder : **Loewe, Heinz, Dr.**
**Berliner Ring 6**
**D-6233 Kelkheim Taunus (DE)**
Erfinder : **Düwel, Dieter, Dr.**
**Frankfurter Strasse 39**
**D-6238 Hofheim am Taunus (DE)**
Erfinder : **Kirsch, Reinhard, Dr.**
**Kurfürstenstrasse 10**
**D-6239 Eppstein/Taunus (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 105 196 B1

Jouve, 18, rue St-Denis, 75001 Paris, France

**Beschreibung**

Die vorliegende Erfindung betrifft neue substituierte Benzolsulfonsäureester, ein Verfahren zu ihrer Herstellung und die Verwendung als Arzneimittel, insbesondere als Anthelminthika.

Benzolsulfonsäureester mit anthelminthischen Wirkungen sind nach DE-OS 26 08 238 (entspr. GB-A 1 570 828) und DE-OS 26 53 766 bekannt.

Es wurden nun neue anthelminthisch wirksame substituierte Benzolsulfonsäureester der Formel I gefunden,

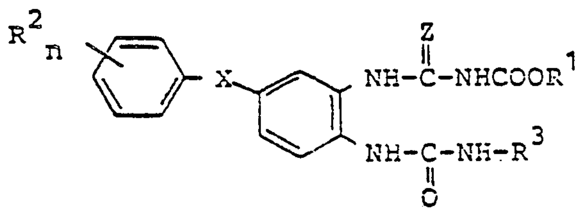

(I)

worin $R^1$ geradkettiges oder verzweigtes Alkyl mit 1-4 C-Atomen, n 1, 2 oder 3 und die einzelnen Substituenten $R^2$ unabhängig voneinander Wasserstoff, Fluor, Chlor, Brom, Jod, Cyano, Nitro, Trifluormethyl, 1,1,2,2-Tetrafluorethoxy, geradkettiges oder verzweigtes Alkyl mit 1-12 C-Atomen, Cycloalkyl mit 3-8 C-Atomen, Alkoxy, Alkylthio, Alkylsulfinyl oder Alkylsulfonyl mit jeweils 1-6 C-Atomen im Alkylrest, Acetyl, Acetamino oder jeweils gegebenenfalls ein- oder zweifach halogensubstituiertes Phenyl, Phenoxy, Phenylthio, Phenylsulfinyl oder Phenylsulfonyl bedeuten, $R^3$ Wasserstoff oder —CO—$R^4$ bedeutet, worin $R^4$ gegebenenfalls durch Halogen substituiertes Alkyl oder Alkoxyalkyl mit jeweils 1-6 C-Atomen in einem Alkylteil oder gegebenenfalls durch Halogen, Methyl oder Methoxy substituiertes Phenyl bedeutet,

X —$SO_2$—O— oder —O—$SO_2$— und

Z =S, =N—COOR$^1$ oder =NH bedeuten, wobei Verbindungen ausgenommen sind, in denen $R^3$ Wasserstoff ist, wenn gleichzeitig

Z =N—COOR$^1$ bedeutet und $R^2$ von 1,1,2,2-Tetrafluoräthoxy, Acetyl, Cycloalkyl und schwefelhaltigen Resten verschieden ist.

Unter den Verbindungen der Formel I sind solche bevorzugt, in denen $R^1$ Methyl und $R^2$ Wasserstoff, Fluor, Chlor, Brom, Jod, Trifluormethyl, 1,1,2,2-Tetrafluorethoxy, geradkettiges oder verzweigtes Alkyl mit 1-6 C-Atomen oder Cycloalkyl mit 3-6 C-Atomen bedeutet, und worin $R^3$ Wasserstoff oder —COR$^4$, worin $R^4$ Alkyl oder Alkoxyalkyl mit jeweils 1-4 C-Atomen in einem Alkylteil oder Phenyl bedeutet, X —$SO_2$—O— oder —O—$SO_2$— und Z =S oder =$NCOOCH_3$ und n 1 oder 2 bedeuten.

Gegenstand der Erfindung ist weiterhin ein Verfahren zur Herstellung von substituierten Benzolsulfonsäureestern der Formel I, dadurch gekennzeichnet, daß man ein Aminoharnstoffderivat der Formel II

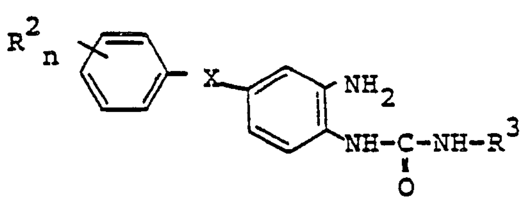

(II)

worin $R^2$, $R^3$, X und n die gleiche Bedeutung haben wie in Formel I, entweder

a) mit einem Isothiocyanatocarboxylat der Formel III,

$$S{=}C{=}N{-}COOR^1 \qquad (III)$$

worin $R^1$ die oben gegebene Bedeutung hat, umsetzt zu einer Verbindung der Formel I, worin Z für S steht, oder

b) mit einem Isothioharnstoffcarboxylat der Formel IV

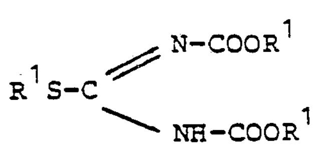

(IV)

worin $R^1$ die oben gegebene Bedeutung hat und die über Sauerstoff und über Schwefel gebundenen Reste $R^1$ unabhängig voneinander gleich oder verschieden sein können in Gegenwart eines Lösungsmittels und einer Säure umsetzt zu einer Verbindung der Formel I worin Z =N—COOR$^1$ bedeutet, oder

c) mit einem Isothioharnstoffcarboxylat der Formel IV zunächst wie nach b) umsetzt zu einer Verbindung der Formel I, worin Z =N—COOR$^1$ bedeutet, und diese Verbindung anschließend mit einer Base verseift zu einer Verbindung der Formel I, worin Z für =NH steht.

Die Aminoverbindungen der allgemeinen Formel II sind neu und werden durch Reduktion von Nitroverbindungen der Formel V erhalten,

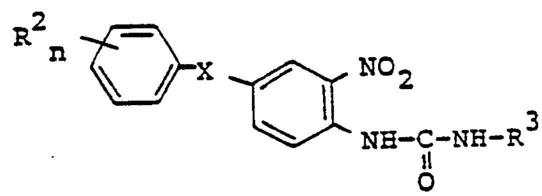
(V)

worin $R^2$, $R^3$, X und n die oben gegebenen Bedeutungen haben. Die Nitroverbindungen der Formel V sind ebenfalls neu und werden aus Sulfochloriden der Formel VI,

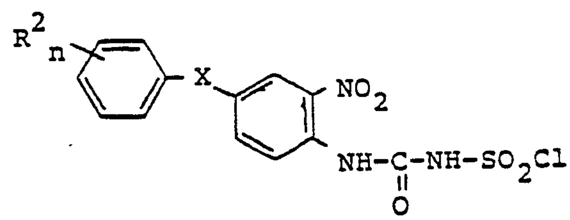
(VI)

worin $R^2$, X und n die oben gegebenen Bedeutungen haben, durch Verseifung (V : $R^3$=H) und gegebenenfalls anschließende Acylierung (V : $R^3$=—CO—$R^4$) erhalten.

Die Sulfochloride der Formel VI sind ebenfalls neu und werden aus Nitroaminoverbindungen der allgemeinen Formel VII

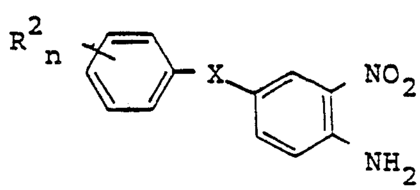
(VII)

worin $R^2$, X und n die oben gegebenen Bedeutungen haben, durch Umsetzung mit N-Chlorsulfonylisocyanat VIII

$$O{=}C{=}N{-}SO_2Cl \qquad (VIII)$$

erhalten.

Nitroamine der Formel VII sind nach DE-OS 24 41 201 und DE-OS 24 41 202 (entsprechend US 3 996 368 und US 3 996 369) bekannt und werden nach den dort angegebenen Methoden oder auf analogem Wege hergestellt.

Wenn X in Formel VII —$SO_2$—O— bedeutet, erfolgt die Herstellung zweckmäßig durch Umsetzung eines gegebenenfalls $R_n^2$-substituierten Benzolsulfonsäurechlorids mit 4-Amino-3-nitrophenol oder dessen Salzen in einem inerten Lösungsmittel wie Aceton, in Gegenwart einer organischen oder anorganischen Base wie Triethylamin oder Natriumhydroxid.

Wenn X in Formel VII —O—$SO_2$— bedeutet, erfolgt die Herstellung zweckmäßig durch Umsetzung eines gegebenenfalls $R_n^2$-substituierten Phenols mit 4-Chlor-3-nitrobenzolsulfonsäurechlorid in einem inerten Lösungsmittel wie Aceton in Gegenwart einer organischen oder anorganischen Base wie Triethylamin oder Natriumhydroxid und anschließenden Chloraustausch in einem inerten Lösungsmittel wie Dioxan mit gasförmigen Ammoniak bei erhöhter Temperatur, vorteilhaft bei 100-140 °C und erhöhtem Druck.

Die Umsetzung der Nitroamine der Formel VII mit N-Chlorsulfonylisocyanat VIII erfolgt in einem aprotischen Lösungsmittel bei — 20 bis + 50 °C, vorzugsweise zwischen 0 und 20 °C. Als Lösungsmittel kommen z. B. Kohlenwasserstoffe wie Hexan, Cyclohexan, Benzol, Toluol, Xylol, Ether wie Diisopropylether, Dioxan, Dimethoxyethan oder Chlorkohlenwasserstoffe wie Methylenchlorid, Chloroform, Tetrachlorkohlenstoff, 1,2-Dichlorethan, Chlorbenzol, ferner Acetonitril in Frage.

In einer bevorzugten Ausführungsform arbeitet man mit einem geringen Überschuß (10-20 %) an VIII in Toluol bei etwa 10 °C.

Die Verseifung der Sulfochloride VI erfolgt durch Verrühren mit Wasser oder einer wässrigen Lösung einer anorganischen oder organischen Säure wie Salzsäure, Schwefelsäure, Phosphorsäure, Salpetersäure, Ameisensäure, Essigsäure, Trifluoressigsäure, p-Toluolsulfonsäure, gegebenenfalls unter Zusatz eines Lösungsmittels wie Methanol, Ethanol, Aceton, bei — 20 bis + 100 °C. Vorzugsweise arbeitet man bei 0 bis 80 °C in 1-2 n HCl. Die bei der Verseifung zunächst gebildete Sulfonsäure ist unbeständig und zerfällt in $SO_3$ und eine Nitroverbindung V mit $R^3$=H. Hieraus kann gegebenenfalls durch Umsetzung mit einem Säureanhydrid $(R^4—CO)_2O$, zweckmäßig in einem inerten Lösungsmittel wie Toluol, Methylenchlorid oder Acetonitril, bei — 20 bis + 50 °C in Gegenwart einer katalytischen Menge einer starken anorganischen oder organischen Säure wie konzentrierter Schwefelsäure, Methansulfonsäure oder Trifluoressigsäure, eine Verbindung V mit $R^3$=—CO—$R^4$ hergestellt werden. In einer bevorzugten Ausführungsform arbeitet man in einem Überschuß des Säureanhydrids bei 0-40 °C und verwendet konzentrierte Schwefelsäure als Katalysator.

Die Nitroverbindungen der Formel V werden durch Reduktion, z. B. mit Raney-Nickel, in einem Lösungsmittel, z. B. Methanol oder Dimethylformamid, bei Normaldruck oder im Autoklaven unter Druck zu den Aminoverbindungen der Formel II hydriert.

Bei der Verfahrensvariante a) erfolgt die Umsetzung der Aminoverbindungen II mit einem Isothiocyanatocarboxylat III mit oder ohne Zusatz eines Lösungsmittels bei — 20 bis + 140 °C.

Als Lösungsmittel kommen z. B. in Betracht : Diisopropylether, Tetrahydrofuran, Dioxan, Dimethoxyethan, Essigsäureethylester, Aceton, Butanon, Toluol, Xylol, Chlorbenzol, Methylenchlorid, Chloroform, Tetrachlorkohlenstoff, 1,2-Dichlorethan, Methanol, Ethanol, Isopropanol, 2-Methoxyethanol. Vorzugsweise wird die Reaktion mit einer äquimolaren Menge oder einem geringen Überschuß (10-20 %) des Isothiocyanatocarboxylats bei 20-50 °C in Dioxan oder Essigester durchgeführt.

Bei der Verfahrensvariante b) erfolgt die Umsetzung der Aminoverbindungen II mit einem Isothioharnstoffcarboxylat IV in Gegenwart eines Lösungsmittels und einer Säure bei 0-120 °C.

Als Lösungsmittel kommen insbesondere polare wie z. B. Wasser, Methanol, Ethanol, Isopropanol, Butanol, 2-Methoxyethanol aber z. B. auch Essigester, Dioxan, Toluol, Chloroform oder deren Gemische in Frage.

Als Säuren können anorganische oder organische Säuren wie Salzsäure, Schwefelsäure, Salpetersäure, Phosphorsäure, Ameisensäure, Essigsäure, Propionsäure, p-Toluolsulfonsäure Verwendung finden. Vorzugsweise wird die Reaktion in Methanol, allein oder im Gemisch mit Essigester, unter Zusatz von Eisessig oder p-Toluolsulfonsäure bei der Rückflußtemperatur des verwendeten Gemisches durchgeführt.

Bei der Verfahrensvariante c) werden solche Verbindungen der allgemeinen Formel I, die nach der Verfahrensvariante b) hergestellt wurden (Z bedeutet $=NCOOR^1$), mit anorganischen oder organischen Basen verseift und decarboxyliert zu Verbindungen der Formel I, in der Z=NH bedeutet. Als Basen kommen z. B. Ammoniak, Alkali- oder Erdalkalicarbonate und -hydroxide wie Kalium-, Bariumcarbonat, Natrium-, Kaliumhydroxid oder Amine wie Methyl-, Ethyl-, Butyl-, Diethyl-, Diisopropyl-, Triethyl-, Ethyldiisopropylamin in einem Lösungsmittel wie Wasser, Methanol, Ethanol, Isopropanol, Essigester, Aceton, Dioxan, Toluol oder deren Gemischen in Betracht. Die Reaktionstemperatur kann dabei zwischen 0 und 120 °C liegen. Beispielsweise arbeitet man mit Butylamin in Methanol zweckmäßig bei der Siedetemperatur der Reaktionsmischung.

Diejenigen Verbindungen der Formel I in denen Z $=NCOOR^1$ oder =NH bedeutet, — Verfahrensvariante b) und c) — können in tautomeren Formen vorliegen

$$(-NH-\underset{\underset{Z}{\|}}{C}-NH-COOR^1 \rightleftharpoons N=\underset{\underset{ZH}{|}}{C}-NH-COOR^1 \rightleftharpoons -NH-\underset{\underset{ZH}{|}}{C}=N-COOR^1$$

in Formel I). Die allgemeine Formel I umfaßt deshalb auch alle Tautomeren und ihre Gemische.

Erfindungsgemäße Verbindungen der Formel I, worin X $—SO_2—O—$ bedeutet, sind zum Beispiel

(Siehe Tabelle Seite 5 ff.)

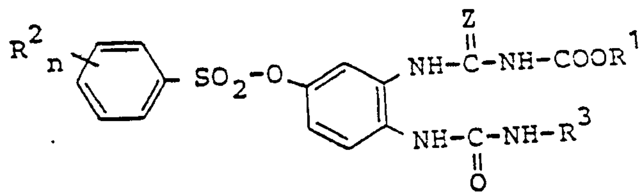

| $R^2_n$ | $R^3$ | $R^1$ | Z |
|---|---|---|---|
| $4-C_6H_{11}$ | H | $CH_3$ | $N-COOCH_3$ |
| $4-C_6H_{11}$ | H | $CH_3$ | NH |
| $4-C_6H_{11}$ | H | $CH_3$ | S |
| $4-C_6H_{11}$ | $CH_3CO$ | $CH_3$ | $N-COOCH_3$ |
| $4-C_6H_{11}$ | $CH_3CO$ | $CH_3$ | S |
| H | $CH_3CO$ | $CH_3$ | $N-COOCH_3$ |
| H | $CH_3CO$ | $CH_3$ | S |
| $2-CF_3$ | $CH_3CO$ | $CH_3$ | $N-COOCH_3$ |
| $2-CF_3$ | $CH_3CO$ | $CH_3$ | S |
| $3-CF_3$ | H | $C_2H_5$ | $N-COOC_2H_5$ |
| $3-CF_3$ | H | $iC_3H_7$ | $N-COOiC_3H_7$ |
| $3-CF_3$ | $C_6H_5CO$ | $CH_3$ | $N-COOCH_3$ |
| $3-CF_3$ | H | $CH_3$ | NH |
| $3-CF_3$ | $CH_3OCH_2CO$ | $CH_3$ | $N-COOCH_3$ |
| $3-CF_3$ | $CH_3OCH_2CO$ | $CH_3$ | S |
| $4-CF_3$ | H | $CH_3$ | NH |
| $4-CF_3$ | H | $CH_3$ | S |
| $4-CF_3$ | $CH_3CO$ | $CH_3$ | $N-COOCH_3$ |
| $4-CF_3$ | $CH_3CO$ | $CH_3$ | S |
| $3,5-(CF_3)_2$ | $CH_3CO$ | $CH_3$ | $N-COOCH_3$ |
| $3,5-(CF_3)_2$ | $CH_3CO$ | $CH_3$ | NH |
| $3-CHF_2CF_2O$ | H | $CH_3$ | NH |
| $3-CHF_2CF_2O$ | $CH_3CO$ | $CH_3$ | $N-COOCH_3$ |
| $3-CHF_2CF_2O$ | $CH_3CO$ | $CH_3$ | S |
| $3-CHF_2CF_2O$ | H | $iC_3H_7$ | S |

(Fortsetzung)

| $R^2_n$ | $R^3$ | $R^1$ | Z |
|---|---|---|---|
| 4-F | H | $CH_3$ | NH |
| 4-F | $CH_3CO$ | $CH_3$ | $N-COOCH_3$ |
| 4-F | $CH_3CO$ | $CH_3$ | S |
| 4-F | $CH_3OCH_2CO$ | $CH_3$ | $N-COOCH_3$ |
| 4-F | $CH_3OCH_2CO$ | $CH_3$ | S |
| 4-Br | $CH_3CO$ | $CH_3$ | $N-COOCH_3$ |
| 4-Br | $CH_3CO$ | $CH_3$ | S |
| 2,5-$Cl_2$ | $CH_3CO$ | $CH_3$ | $N-COOCH_3$ |
| 2,5-$Cl_2$ | $CH_3CO$ | $CH_3$ | S |
| 2,4-$Cl_2$ | H | $CH_3$ | S |
| 3-Br,4-F | H | $CH_3$ | S |
| 3,4-$F_2$ | H | $CH_3$ | NH |
| 3,4-$F_2$ | H | $CH_3$ | S |
| 3,4-$F_2$ | H | $C_4H_9$ | S |
| 3,4-$Br_2$ | H | $CH_3$ | S |
| 2-Cl,4-Br | H | $CH_3$ | S |
| 2-Cl | H | $CH_3$ | S |
| 2-Br | H | $CH_3$ | S |
| 2-F | H | $CH_3$ | S |
| 4-J | H | $CH_3$ | NH |
| 4-J | H | $CH_3$ | S |
| 3-F | H | $CH_3$ | S |
| 4-$CH_3O$ | $CH_3CO$ | $CH_3$ | $N-COOCH_3$ |
| 4-$CH_3O$ | $CH_3CO$ | $CH_3$ | S |
| 4-$CH_3S$ | H | $CH_3$ | $N-COOCH_3$ |
| 4-$CH_3S$ | H | $CH_3$ | S |
| 4-$CH_3SO$ | H | $CH_3$ | $N-COOCH_3$ |
| 4-$CH_3SO$ | H | $CH_3$ | S |
| 4-$CH_3SO_2$ | H | $CH_3$ | $N-COOCH_3$ |
| 4-$CH_3SO_2$ | H | $CH_3$ | S |
| 3-$CH_3SO_2$ | H | $CH_3$ | $N-COOCH_3$ |
| 3-$CH_3SO_2$ | H | $CH_3$ | NH |
| 3-$CH_3SO_2$ | H | $CH_3$ | S |
| 4-$C_6H_5O$ | H | $CH_3$ | S |
| 4-$C_6H_5S$ | H | $CH_3$ | $N-COOCH_3$ |

(Fortsetzung)

| $R^2_n$ | $R^3$ | $R^1$ | Z |
|---|---|---|---|
| $4\text{-}C_6H_5S$ | H | $CH_3$ | NH |
| $4\text{-}C_6H_5S$ | H | $CH_3$ | S |
| $4\text{-}C_6H_5SO$ | H | $CH_3$ | $N\text{-}COOCH_3$ |
| $4\text{-}C_6H_5SO$ | H | $CH_3$ | S |
| $4\text{-}C_6H_5SO_2$ | H | $CH_3$ | $N\text{-}COOCH_3$ |
| $4\text{-}C_6H_5SO_2$ | H | $CH_3$ | S |
| $4\text{-}C_6H_5CH_2$ | H | $CH_3$ | S |
| $2,3\text{-}Cl_2$ | H | $CH_3$ | S |
| 3-CN | H | $CH_3$ | S |
| $3\text{-}NO_2$ | H | $CH_3$ | S |
| $4\text{-}F\text{-}C_6H_4$ | H | $CH_3$ | S |
| $4\text{-}Cl\text{-}C_6H_4$ | H | $CH_3$ | S |
| $4\text{-}Br\text{-}C_6H_4$ | H | $CH_3$ | S |

Erfindungsgemäße Verbindungen der Formel I, worin X —O—$SO_2$— bedeutet, sind zum Beispiel

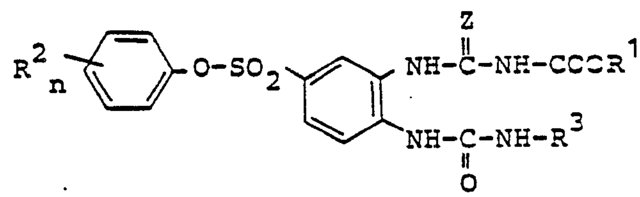

| $R^2_n$ | $R^3$ | $R^1$ | Z |
|---|---|---|---|
| H | H | $CH_3$ | NH |
| H | $C_2H_5CO$ | $CH_3$ | S |
| H | $C_2H_5CO$ | $C_2H_5$ | S |
| H | $C_2H_5CO$ | $iC_3H_7$ | S |
| H | $CH_3OCH_2CO$ | $CH_3$ | $N\text{-}COOCH_3$ |
| H | $CH_3OCH_2CO$ | $CH_3$ | S |
| H | $C_6H_5CO$ | $CH_3$ | S |
| $4\text{-}C_2H_5$ | $CH_3CO$ | $CH_3$ | $N\text{-}COOCH_3$ |
| $4\text{-sec.}C_4H_9$ | $CH_3CO$ | $CH_3$ | $N\text{-}COOCH_3$ |
| 4-Cl | H | $CH_3$ | NH |
| 4-Cl | H | $CH_3$ | S |
| 4-Br | H | $CH_3$ | S |
| 4-J | H | $CH_3$ | S |
| $3,4\text{-}Cl_2$ | H | $CH_3$ | S |
| $3\text{-}CF_3$ | $C_3H_7CO$ | $CH_3$ | $N\text{-}COOCH_3$ |
| $3\text{-}CF_3$ | $C_3H_7CO$ | $CH_3$ | S |

(Fortsetzung)

| $R^2_n$ | $R^3$ | $R^1$ | Z |
|---|---|---|---|
| $3-CF_3$ | $C_3H_7CO$ | $C_2H_5$ | S |
| $3-CF_3$ | $CH_3OCH_2CO$ | $CH_3$ | $N-COOCH_3$ |
| $3-CF_3$ | $CH_3OCH_2CO$ | $CH_3$ | NH |
| $3-CF_3$ | $CH_3OCH_2CO$ | $CH_3$ | S |
| $3-CN$ | H | $CH_3$ | S |
| $4-CN$ | H | $CH_3$ | S |
| $3-OCH_3$ | H | $CH_3$ | S |
| $4-OCH_3$ | H | $CH_3$ | S |
| $3,4-(OCH_3)_2$ | H | $CH_3$ | S |
| $3-NO_2$ | H | $CH_3$ | S |
| $4-(CH_3CONH)$ | H | $CH_3$ | S |
| $4-C_6H_5-CH_2$ | H | $CH_3$ | S |
| $4-C_6H_5O$ | H | $CH_3$ | S |
| $4-C_6H_5S$ | H | $CH_3$ | $N-COOCH_3$ |
| $4-C_6H_5S$ | H | $CH_3$ | S |
| $4-C_6H_5SO$ | H | $CH_3$ | S |
| $4-C_6H_5SO_2$ | H | $CH_3$ | S |

Die neuen substituierten Benzolsulfonsäureester der Formel I gemäß der Erfindung sind wertvolle Chemotherapeutika und eignen sich zur Bekämpfung von parasitären Erkrankungen von Mensch und Tier.

Sie sind besonders wirksam gegen eine große Anzahl von Helminthen, z. B. Haemonchus, Trichostrongylus, Ostertagia, Cooperia, Chabertia, Strongyloides, Oesophagostomum, Hyostrongylus, Ancylostoma, Ascaris und Heterakis sowie Fasciola. Besonders ausgeprägt ist die Wirksamkeit gegenüber Magen-Darmstrongyliden, Lungenwürmern und Leberegeln, von denen vor allem Haus- und Nutztiere befallen werden. Deshalb werden die Verbindungen gemäß der Erfindung insbesondere in Tierarzneimitteln verwendet.

Die Verbindungen der Formel I werden je nach Lage des Falles in Dosierungen zwischen 0,1 und 50 mg pro kg Körpergewicht 1 bis 14 Tage lang verabreicht.

Zur oralen Applikation kommen Tabletten, Dragees, Kapseln, Boli, Pulver, Granulate oder Pasten in Betracht, welche die Wirkstoffe zusammen mit üblichen Hilfs- oder Trägerstoffen wie Stärke, Cellulosepulver, Talcum, Magnesiumstearat, Zucker, Gelatine, Calciumcarbonat, feinverteilter Kieselsäure, Carboxymethylcellulose oder ähnlichen Stoffen enthalten.

Die Verfahrensprodukte sind nicht nur oral appliziert ausgezeichnet wirksam, sondern können auch parenteral appliziert werden.

Zur parenteralen Applikation kommen Lösungen in Betracht, z. B. ölige Lösungen, die unter Verwendung von Sesamöl, Olivenöl oder synthetischen Triglyceriden, ggfs. mit einem Zusatz wie z. B. von Tokopherol als Antioxydans und/oder unter Verwendung von grenzflächenaktiven Stoffen wie Sorbitanfettsäureester hergestellt werden. Daneben kommen wäßrige Suspensionen in Betracht, die unter Verwendung von äthoxylierten Sorbitan-Fettsäureestern, und/oder unter Zusatz von Verdickungsmitteln, wie Polyäthylenglykol oder Carboxymethylcellulose, hergestellt werden.

Die Konzentrationen der Wirkstoffe gemäß der Erfindung in den damit hergestellten Präparaten liegen vorzugsweise für den Gebrauch als Veterinärarzneimittel zwischen 0,5 und 25 Gewichtsprozent ; für den Gebrauch als Humanarzneimittel liegen die Konzentrationen der Wirkstoffe vorzugsweise zwischen 20 und 80 Gewichtsprozent.

Die neuen Amino-harnstoffderivate der Formel II und die neuen Nitroverbindungen der Formel V sind ebenfalls anthelminthisch wirksam. Außerdem können sie ebenso wie die neuen Sulfochloride der Formel VI als Zwischenprodukte zur Herstellung von Anthelminthika dienen, wie vorstehend beschrieben.

Beispiel 1 : Verfahren A

1-Methoxycarbonyl-3-(5-phenoxysulfonyl-2-ureido-phenyl)-thioharnstoff (Formel I)

Zu 23,5 g 5-Phenoxysulfonyl-2-ureido-analin in 100 ml Dioxan werden unter Rühren 25 ml Methoxycarbonylisothiocyanat zugetropft, wobei die Temperatur bis auf ca. 35 °C ansteigt. Man rührt ohne Kühlung vier Stunden weiter, destilliert das Dioxan ab, verrührt mit Diisopropylether und saugt ab.

Nach Kristallisation aus Methanol, Fp. 190 °C Zers.

Beispiel 2 : Verfahren A

1-[2-(3-Acetylureido)-5-phenoxysulfonyl-phenyl]-3-methoxycarbonyl-thioharnstoff (Formel I)

17,5 g 2-(3-Acetylureido)-5-phenoxysulfonyl-anilin und 15 ml Methoxycarbonylisothiocyanat werden in 150 ml Dioxan fünf Minuten auf 80 °C erwärmt, anschließend ohne Heizung weitergerührt. Nach dem langsamen Abkühlen auf Raumtemperatur wird das Dioxan bei vermindertem Druck abdestilliert. Der Rückstand wird in Dimethylformamid gelöst, gekohlt, eingeengt und dann mit Methanol versetzt. Nach dem Abkühlen wird die Fällung abgesaugt und mit Methanol und Diisopropylether gewaschen, Fp. 215 °C Zers.

Beispiel 3

5-Phenoxysulfonyl-2-ureido-anilin (Formel II)

33,7 g 5-Phenoxysulfonyl-2-ureido-nitrobenzol werden in 250 ml Methanol und 250 ml 2-Methoxyethanol mit einer katalytischen Menge Raney-Nickel bei Normaldruck hydriert. Nach beendeter Wasserstoffauhnahme wird der Katalysator abgesaugt, mit Dimethylformamid gewaschen und die Lösung unter vermindertem Druck eingeengt, mit Methanol zur Kristallisation gebracht, abgesaugt und mit Methanol gewaschen, Fp. 180 °C Zers.

Beispiel 4

5-Phenoxysulfonyl-2-ureido-nitrobenzol (Formel V)

Zu 88,2 g 2-Amino-5-phenoxysulfonyl-nitrobenzol in 750 ml Toluol werden bei + 10 °C 30 ml N-Chlorsulfonylisocyanat gelöst in 70 ml Toluol rasch zugetropft. Man rührt eine Stunde unter Kühlung weiter, saugt die Fällung ab, wäscht mit Diisopropylether nach und trocknet im Exsiccator über Kaliumhydroxid. 88 g des entstandenen 2-(3-Chlorsulfonylureido)-5-phenoxysulfonyl-nitrobenzol werden unter Rühren und Kühlung in 800 ml 1n-HCl eingetragen. Nach 30 Minuten wird die klare Lösung kurz auf 80 °C erwärmt (Fällung) und sich dann selbst überlassen. Nach dem Abkühlen wird abgesaugt und mit Wasser neutral gewaschen, Fp. 190 °C.

Beispiel 5

2-(3-Acetylureido)-5-phenoxysulfonyl-nitrobenzol (Formel V)

50,5 g 5-Phenoxysulfonyl-2-ureido-nitrobenzol in 250 ml Acetanhydrid werden unter Rühren mit 1 ml konzentrierter Schwefelsäure versetzt. Es entsteht eine klare Lösung und kurz darauf eine Fällung. Man rührt zwei Stunden nach, saugt ab und wäscht nacheinander mit Eisessig und Diisopropylether, Fp. 198 °C Zers.

Beispiel 6

2-(3-Acetylureido)-5-phenoxysulfonyl-anilin (Formel II)

48 g 2-(3-Acetylureido)-5-phenoxysulfonyl-nitrobenzol werden in 300 ml Methanol und 300 ml 2-Methoxyethanol mit einer katalytischen Menge Raney-Nickel bei Normaldruck hydriert. Nach beendeter Wasserstoffaufnahme wird der Katalysator abgesaugt, mit Dimethylformamid gewaschen und die Lösung unter vermindertem Druck eingeengt. Der Rückstand wird mit Methanol heiß verrührt, kalt abgesaugt und mit Methanol und Diisopropylether gewaschen, Fp. 202 °C Zers.

Beispiel 7 : Verfahren A

1-Methoxycarbonyl-3-[5-(3-trifluormethylphenyl-sulfonyloxy)-2-ureido-phenyl]-thioharnstoff (Formel I)

Zu 18,7 g 5-(3-Trifluormethylphenylsulfonyloxy)-2-ureido-anilin in 300 ml Dioxan werden unter Rühren 12 ml Methoxycarbonyl-isothiocyanat zugetropft, wobei die Temperatur bis auf ca. 40 °C steigt. Man rührt ohne Kühlung vier Stunden weiter, gibt 60 ml Diisopropylether zu und saugt die Fällung ab.

Nach Waschen mit Diisopropylether wird unter Zusatz von Aktivkohle aus Methanol/Diisopropylether umkristallisiert, Fp. 190 °C Zers.

Beispiel 8 : Verfahren B

N-[2-(3-Acetylureido)-5-(3-trifluormethylphenylsulfonyloxy)-phenyl]-N',N"-bismethoxycarbonyl-guanidin (Formel I)

14,6 2-(3-Acetylureido)-5-(3-trifluormethylphenylsulfonyloxy)-anilin, 12,4 g N,N'-Bismethoxycarbonyl-S-methylisothioharnstoff und 75 mg p-Toluolsulfonsäure werden zwei Stunden unter Rückfluß gerührt. Nach dem Abkühlen wird unter vermindertem Druck eingeengt, aus Isopropanol umkristallisiert und mit Essigester kalt verrührt, abgesaugt und gewaschen, Fp. 189 °C Zers.

Beispiel 9

5-(3-Trifluormethylphenylsulfonyloxy)-2-ureidoanilin (Formel II)

61,5 g 5-(3-Trifluormethylphenylsulfonyloxy)-2-ureidonitrobenzol werden in 200 ml Methanol und 200 ml 2-Methoxyethanol mit einer katalytischen Menge Raney-Nickel bei Normaldruck hydriert. Nach beendeter Wasserstoffaufnahme wird der Katalysator abgesaugt, mit Methanol gewaschen und die Lösung unter vermindertem Druck eingeengt, mit 1 l Wasser versetzt, abgesaugt, mit Wasser gewaschen und über NaOH getrocknet, Fp. 172 °C Zers.

Beispiel 10

5-(3-Trifluormethylphenylsulfonyloxy)-2-ureido-nitrobenzol (Formel V)

Zu 108 g 2-Amino-5-(3-trifluormethylphenylsulfonyloxy)-nitrobenzol in 800 ml Toluol werden bei + 10 °C 30 ml N-Chlorsulfonyl-isocyanat zugetropft. Man rührt zwei Stunden unter Kühlung weiter und versetzt mit 500 ml Petrolether. Das entstandene 2-(3-Chlorsulfonylureido)-5-(3-trifluormethylphenylsulfonyloxy)-nitrobenzol wird abgesaugt, mit Petrolether gewaschen und über Kaliumhydroxid getrocknet, Fp. 137 °C Zers. 150 g dieses Sulfochlorids werden unter Rühren und Eiskühlung in 2 l 1n-HCl eingetragen. Man rührt 2 Stunden unter Kühlung, 5 Stunden bei Raumtemperatur, saugt die Fällung am nächsten Tag ab, wäscht mit Wasser neutral und trocknet, Fp. 152 °C.

Beispiel 11

2-(3-Acetylureido)-5-(3-trifluormethylphenylsulfonyloxy)-nitrobenzol (Formel V)

60 g 5-(3-Trifluormethylphenylsulfonyloxy)-2-ureidonitrobenzol in 200 ml Acetanhydrid werden unter Rühren mit 1 ml konzentrierter Schwefelsäure versetzt. Es entsteht zunächst eine klare Lösung und die Temperatur steigt auf ca. 40 °C an. Man rührt vier Stunden nach, saugt die entstehende Fällung ab und wäscht mit Diisopropylether und Petrolether nach, Fp. 196 °C.

Beispiel 12

2-(3-Acetylureido)-5-(3-trifluormethylphenylsulfonyloxy)-anilin (Formel II)

59 g 2-(3-Acetylureido)-5-(3-trifluormethylphenylsulfonyloxy)-nitrobenzol werden in 200 ml Methanol und 200 ml 2-Methoxyethanol mit einer katalytischen Menge Raney-Nickel bei Normaldruck hydriert. Nach beendeter Wasserstoffaufnahme wird der Katalysator abgesaugt, mit warmem 2-Methoxyethanol gewaschen und die Lösung unter vermindertem Druck eingeengt. Der Rückstand wird mit Wasser versetzt, abgesaugt, mit Wasser gewaschen und über Natriumhydroxid getrocknet, Fp. 182 °C.

In Analogie zu den Beispielen 1 und 2 werden die folgenden erfindungsgemäßen Verbindungen der Formel I, in der X —O—$SO_2$— und $R^1$ Methyl bedeutet (= Formel I a) hergestellt :

(Siehe Tabelle Seite 11 f.)

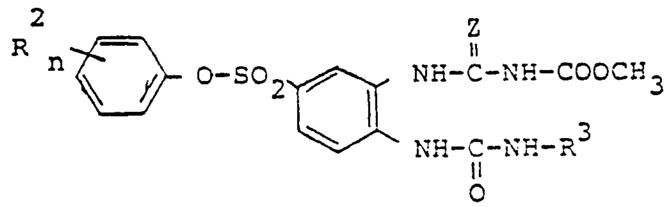

(Ia)

| Beispiel | $R^2_n$ | $R^3$ | Z | Fp.(°C) (Z=Zersetzung) |
|---|---|---|---|---|
| 13 | H | $COCH_3$ | $N-COOCH_3$ | 180 (Z) |
| 14 | $4-CH_3$ | H | S | 198 (Z) |
| 15 | $4-CH_3$ | $COCH_3$ | S | 215 (Z) |
| 16 | $4-CH_3$ | $COCH_3$ | $N-COOCH_3$ | 213 (Z) |
| 17 | $4-C_2H_5$ | H | S | 150 (Z) |
| 18 | $4-C_2H_5$ | $COCH_3$ | S | 205 (Z) |
| 19 | $4-C_2H_5$ | $COCH_3$ | $N-COOCH_3$ | 201 (Z) |
| 20 | $4-i-C_3H_7$ | H | S | 180 (Z) |
| 21 | $4-i-C_3H_7$ | $COCH_3$ | S | 210 (Z) |
| 22 | $4-i-C_3H_7$ | $COCH_3$ | $N-COOCH_3$ | 198 (Z) |
| 23 | $4-s-C_4H_9$ | H | S | 145 |
| 24 | $4-s-C_4H_9$ | $COCH_3$ | S | 193 (Z) |
| 25 | $4-t-C_4H_9$ | H | S | 154 (Z) |
| 26 | $4-t-C_4H_9$ | $COCH_3$ | S | 225 (Z) |
| 27 | $4-t-C_4H_9$ | $COCH_3$ | $N-COOCH_3$ | 208 (Z) |
| 28 | $3-CF_3$ | H | S | 190 (Z) |
| 29 | $3-CF_3$ | $CH_3CO$ | S | 230 (Z) |
| 30 | $3-CF_3$ | $CH_3CO$ | $N-COOCH_3$ | 190 (Z) |
| 31 | $2,4-Cl_2$ | H | S | 160 (Z) |
| 32 | $2,4-Cl_2$ | $CH_3CO$ | S | 225 (Z) |
| 33 | $2,4-Cl_2$ | $CH_3$ | $N-COOCH_3$ | 189 (Z) |
| 34 | $2,3-Cl_2$ | H | S | 195 (Z) |
| 35 | $4-C_6H_5$ | H | S | 120 (Z) |
| 36 | $2-C_6H_{11}$ | H | S | 201 (Z) |
| 37 | $2-C_6H_{11}, 4-CH_3$ | H | S | 200 (Z) |
| 38 | $4-C_6H_{11}$ | H | S | 205 (Z) |
| 39 | 4-F | H | S | 151 (Z) |
| 40 | 3-F | H | S | 170 (Z) |
| 41 | 2-F | H | S | 165 (Z) |

Die Ausgangsprodukte der Formel II, in denen X für —O—$SO_2$— steht (= Formel II a), für die Beispiele 13-41 werden in Analogie zu den Beispielen 3-6 aus den entsprechendne Nitroaminoverbindungen der Formel VII a über die Nitro-ureidoverbindungen V a hergestellt (X in Formel V und VII = —O—$SO_2$—) :

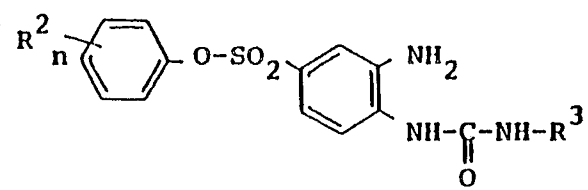

II a

| Beispiel | $R^2_n$ | Verbindung der Formel VIIa, Fp. [°C] | Verbindung der Formel Va, Fp. [°C] | $R^3$ | Verbindung der Formel II a, Fp. [°C] |
|---|---|---|---|---|---|
| 42 | H | 97 | 190 | H | 180 (Z) |
| 43 | H | | 198 (Z) | $CH_3CO$ | 202 (Z) |
| 44 | 4-$CH_3$ | 137 | 172 | H | 184 (Z) |
| 45 | 4-$CH_3$ | | 215 (Z) | $CH_3CO$ | 195 |
| 46 | 4-$C_2H_5$ | 98 | 175 (Z) | H | 178 (Z) |
| 47 | 4-$C_2H_5$ | | 173 | $CH_3CO$ | 160 |
| 48 | 4-i-$C_3H_7$ | 94 | 192 | H | 184 (Z) |
| 49 | 4-i-$C_3H_7$ | | 199 | $CH_3CO$ | 190 |
| 50 | 4-s-$C_4H_9$ | 100 | 135 | H | 170 (Z) |
| 51 | 4-s-$C_4H_9$ | | 186 | $CH_3CO$ | 160 (Z) |

(Fortsetzung)

| Beispiel | $R^2_n$ | Verbindung der Formel VIIa, Fp. [°C] | Verbindung der Formel Va, Fp. [°C] | $R^3$ | Verbindung der Formel IIa, Fp. [°C] |
|---|---|---|---|---|---|
| 52 | 4-t-$C_4H_9$ | 148 | 198 | H | 172 (Z) |
| 53 | 4-t-$C_4H_9$ | | 214 | $CH_3CO$ | 198 |
| 54 | 3-$CF_3$ | 130 | 198 | H | 161 (Z) |
| 55 | 3-$CF_3$ | | 183 | $CH_3CO$ | 203 |
| 56 | 2,4-$Cl_2$ | 155 | 167 (Z) | H | 165 (Z) |
| 57 | 2,4-$Cl_2$ | | 192 (Z) | $CH_3CO$ | 185 |
| 58 | 2,3-$Cl_2$ | 176 | 218 | H | 172 (Z) |
| 59 | 4-$C_6H_5$ | 152 | 188 (Z) | H | — |
| 60 | 2-$C_6H_{11}$ | Öl | 168 | H | 143 (Z) |
| 61 | 2-$C_6H_{11}$, 4-$CH_3$ | Öl | 129 (Z) | H | Harz |
| 62 | 4-$C_6H_{11}$ | 120 | — | H | 270 (Z) |
| 63 | 4-F | 110 | — | H | 154 (Z) |
| 64 | 3-F | 116 | 171 | H | 254 (Z) |
| 65 | 2-F | 125 | 120 (Z) | H | 278 (Z) |

In Analogie zu den Beispielen 7 und 8 werden z. B. die folgenden erfindungsgemäßen Verbindungen der Formel I, in der X $-SO_2-O-$ und $R^1$ Methyl bedeutet (= Formel I b), hergestellt :

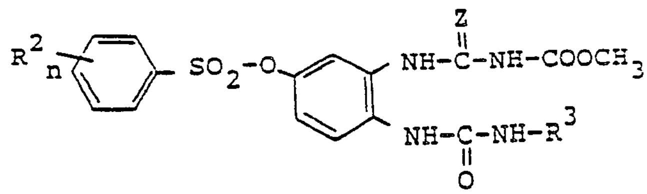

(Ib)

| Beispiel | $R^2_n$ | $R^3$ | Z | Fp. (°C) (Z=Zersetzung) |
|---|---|---|---|---|
| 66 | H | H | S | 200 (Z) |
| 67 | $4-CH_3$ | H | S | 180 (Z) |
| 68 | $4-CH_3$ | $CH_3CO$ | S | 205 (Z) |
| 69 | $4-CH_3$ | $CH_3CO$ | $N-COOCH_3$ | 194 |
| 70 | $4-C_2H_5$ | H | S | 140 (Z) |
| 71 | $4-C_2H_5$ | $CH_3CO$ | S | 205 (Z) |
| 72 | $4-C_2H_5$ | $CH_3CO$ | $N-COOCH_3$ | 173 (Z) |
| 73 | $4-i-C_3H_7$ | H | S | 165 (Z) |
| 74 | $4-i-C_3H_7$ | $CH_3CO$ | S | 222 (Z) |
| 75 | $4-i-C_3H_7$ | $CH_3CO$ | $N-COOCH_3$ | 194 (Z) |
| 76 | $4-s-C_4H_9$ | H | S | 140 |
| 77 | $4-s-C_4H_9$ | $CH_3CO$ | S | 215 (Z) |
| 78 | $4-s-C_4H_9$ | $CH_3CO$ | $N-COOCH_3$ | 175 (Z) |
| 79 | $4-t-C_4H_9$ | H | S | 170 (Z) |
| 80 | $4-t-C_4H_9$ | $CH_3CO$ | S | 228 (Z) |
| 81 | $4-t-C_4H_9$ | $CH_3CO$ | $N-COOCH_3$ | 202 |
| 82 | $3-CF_3$ | $CH_3CO$ | S | 220 (Z) |
| 83 | $4-Cl$ | H | S | 188 (Z) |
| 84 | $4-Cl$ | $CH_3CO$ | S | 223 (Z) |
| 85 | $4-Cl$ | $CH_3CO$ | $N-COOCH_3$ | 145 (Z) |
| 86 | $2-CF_3$ | H | S | 173 (Z) |
| 87 | $3,5-(CF_3)_2$ | H | S | 191 (Z) |
| 88 | $3,5-(CF_3)_2$ | $CH_3CO$ | S | 210 (Z) |
| 89 | $3-CHF_2CF_2O$ | H | S | 147 (Z) |
| 90 | $3-CHF_2CF_2O$ | H | $N-COOCH_3$ | 170 (Z) |
| 91 | $4-F$ | H | S | 193 (Z) |
| 92 | $4-Br$ | H | S | 186 (Z) |
| 93 | $2,5-Cl_2$ | H | S | 179 (Z) |
| 94 | $4-OCH_3$ | H | S | 120 |
| 95 | $3-CH_3CO$ | H | S | 176 (Z) |
| 96 | $3-CH_3CO$ | H | $N-COOCH_3$ | 168 (Z) |
| 97 | $2,4,6-(i-C_3H_7)_3$ | H | S | 191 (Z) |
| 98 | $3,5-(CF_3)_2$ | $CH_3CO$ | $N-COOCH_3$ | 195 (Z) |
| 99 | $3-CH_3SO_2$ | H | $N-COOCH_3$ | 142 (Z) |
| 100 | $4-CH_3SO_2$ | H | $N-COOCH_3$ | 190 (Z) |

Die Ausgangsprodukte der Formel II, in denen X für $—SO_2—O—$ steht (= Formel II b), für die Beispiele 66-100 werden in Analogie zu den Beispielen 9-12 aus den entsprechenden Nitroaminoverbindungen der Formel VII b über die Nitroureidoverbindungen V b hergestellt (X in Formel V und VII = $—SO_2—O—$) :

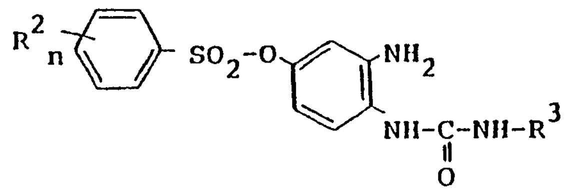

(IIb)

| Beispiel | $R^2_n$ | Verbindung der Formel VII b, Fp. [°C] | Verbindung der Formel Vb, Fp. [°C] | $R^3$ | Verbindung der Formel II b, Fp. [°C] |
|---|---|---|---|---|---|
| 101 | H | 149 | 181 | H | 154 (Z) |
| 102 | H | | - | $CH_3CO$ | |
| 103 | $4-CH_3$ | 155 | 180 | H | 162 (Z) |
| 104 | $4-CH_3$ | | 179 | $CH_3CO$ | 195 |
| 105 | $4-C_2H_5$ | 120 | 160 | H | 170 (Z) |
| 106 | $4-C_2H_5$ | | 185 | $CH_3CO$ | 160 |
| 107 | $4-i-C_3H_7$ | 105 | 160 | H | 150 (Z) |
| 108 | $4-i-C_3H_7$ | | 218 | $CH_3CO$ | 182 (Z) |
| 109 | $4-s-C_4H_9$ | 70 - 72 | 112 | H | 145 (Z) |

| Beispiel | $R^2_n$ | Verbindung der Formel VIIb, Fp. [°C] | Verbindung der Formel Vb, Fp. [°C] | $R^3$ | Verbindung der Formel II b, Fp. [°C] |
|---|---|---|---|---|---|
| 110 | 4-s-$C_4H_9$ | | 195 | $CH_3CO$ | 165 (Z) |
| 111 | 4-t-$C_4H_9$ | 125 | 153 | H | 173 (Z) |
| 112 | 4-t-$C_4H_9$ | | 235 (Z) | $CH_3CO$ | 203 (Z) |
| 113 | 3-$CF_3$ | 132 | 152 | H | 172 (Z) |
| 114 | 3-$CF_3$ | | 196 | $CH_3CO$ | 182 |
| 115 | 4-Cl | 147 | 177 | H | 166 (Z) |
| 116 | 4-Cl | | 188 | $CH_3CO$ | 189 (Z) |
| 117 | 2-$CF_3$ | 132 | 162 (Z) | H | – |
| 118 | 3,5-$(CF_3)_2$ | 154 | 194 | H | 171 (Z) |
| 119 | 3,5-$(CF_3)_2$ | | 153 | $CH_3CO$ | 201 (Z) |
| 120 | 3-$CHF_2CF_2O$ | Harz | Harz | H | – |
| 121 | 4-F | 161 | 178 | H | 158 (Z) |
| 122 | 4-F | | 185 | $CH_3CO$ | – |
| 123 | 4-Br | 165 | 172 | H | 170 (Z) |
| 124 | 2,5-$Cl_2$ | 189 | 182 | H | – |
| 125 | 4-$OCH_3$ | 102 | 178 (Z) | H | Harz |
| 126 | 3-$CH_3CO$ | 142 | – | H | 162 |
| 127 | 2,4,6-$(i-C_3H_7)_3$ | 152 | 145 (Z) | H | – |
| 128 | 3-$CH_3SO_2$ | 148 | 148 (Z) | H | 180 (Z) |
| 129 | 4-$CH_3SO_2$ | 181 | 190 (Z) | H | 280 (Z) |

**Patentansprüche** (für die Vertragsstaaten : BE, CH, DE, FR, GB, IT, LI, LU, NL, SE)

1. Benzolsulfonsäureester der Formel I

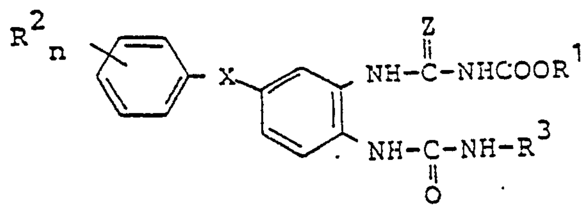

(I)

worin $R^1$ geradkettiges oder verzweigtes Alkyl mit 1-4 C-Atomen, n 1, 2 oder 3 und die einzelnen Substituenten $R^2$ unabhängig voneinander Wasserstoff, Fluor, Chlor, Brom, Jod, Cyano, Nitro, Trifluormethyl, 1,1,2,2-Tetrafluorethoxy, geradkettiges oder verzweigtes Alkyl mit 1-12 C-Atomen, Cycloalkyl mit 3-8 C-Atomen, Alkoxy, Alkylthio, Alkylsulfinyl oder Alkylsulfonyl mit jeweils 1-6 C-Atomen im Alkylrest, Acetyl, Acetamino oder jeweils gegebenenfalls ein- oder zweifach halogensubstituiertes Phenyl, Phenoxy, Phenylthio, Phenylsulfinyl oder Phenylsulfonyl bedeuten, $R^3$ Wasserstoff oder —CO—$R^4$ bedeutet, worin $R^4$ gegebenenfalls durch Halogen substituiertes Alkyl oder Alkoxyalkyl mit jeweils 1-6 C-Atomen in einem Alkylteil oder gegebenenfalls durch Halogen, Methyl oder Methoxy substituiertes Phenyl bedeutet, X —$SO_2$—O— oder —O—$SO_2$— und Z =S, N =COOR$^1$ oder =NH bedeuten, wobei Verbindungen ausgenommen sind, in denen $R^3$ Wasserstoff ist, wenn gleichzeitig Z =N—OOOR$^1$ bedeutet und $R^2$ von 1,1,2,2-Tetrafluoräthoxy, Acetyl, Cycloalkyl und schwefelhaltigen Resten verschieden ist.

2. Verbindungen gemäß Formel I in Anspruch 1, worin $R^1$ Methyl und $R^2$ Wasserstoff, Fluor, Chlor, Brom, Jod, Trifluormethyl, 1,1,2,2-Tetrafluorethoxy, geradkettiges oder verzweigtes Alkyl mit 1-6 C-Atomen oder Cycloalkyl mit 3-6 C-Atomen bedeutet, und worin $R^3$ Wasserstoff oder —COR$^4$, worin $R^4$ Alkyl oder Alkoxyalkyl mit jeweils 1-4 C-Atomen in einem Alkylteil oder Phenyl bedeutet, X —$SO_2$—O— oder —O—$SO_2$— und Z =S oder =$NCOOCH_3$ und n 1 oder 2 bedeuten.

3. Verbindungen gemäß Formel I in Anspruch 1, dadurch gekennzeichnet, daß $R^1$ Methyl, n 1, $R^2$ 3-Trifluormethyl, $R^3$ Wasserstoff, X —$SO_2$—O— und Z =N—$COOCH_3$ bedeutet.

4. Verfahren zur Herstellung von substituierten Benzolsulfonsäureestern der Formel I in Anspruch 1, dadurch gekennzeichnet, daß man ein Amino-harnstoffderivat der Formel

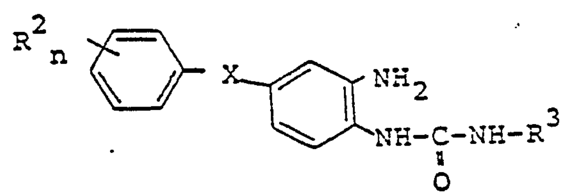

(II)

worin $R^2$, n und $R^3$ die zu Formel I in Anspruch 1 angegebenen Bedeutungen haben, entweder

a) mit einem Isothiocyanatocarboxylat der Formel S=C=N—COOR$^1$ (III), worin $R^1$ die oben gegebene Bedeutung hat, umsetzt zu einer Verbindung der Formel I, worin Z für =S steht, oder

b) mit einem Isothioharnstoffcarboxylat der Formel

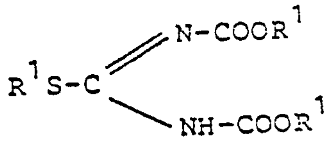

(IV)

worin $R^1$ die oben gegebene Bedeutung hat und die über Sauerstoff und über Schwefel gebundenen Reste $R^1$ unabhängig voneinander gleich oder verschieden sein können in Gegenwart eines Lösungsmittels und einer Säure umsetzt zu einer Verbindung der Formel I, worin Z =N—COOR$^1$ bedeutet, oder

c) mit einem Isothioharnstoffcarboxylat der Formel IV zunächst wie nach b) umsetzt zu einer Verbindung der Formel I, worin Z =N—COOR$^1$ bedeutet, und diese Verbindung anschließend mit einer Base verseift zu einer Verbindung der Formel I, worin Z für =NH steht.

5. Mittel zur Bekämpfung von Helminthen, dadurch gekennzeichnet, daß es einen substituierten Benzolsulfonsäureester der Formel I in Anspruch 1, 2 oder 3 in Mischung mit pharmazeutisch üblichen Trägerstoff enthält oder in Mischung mit einem üblichen Futterstoff.

6. Verwendung eines substituierten Benzolsulfonsäureesters der Formel I in Anspruch 1, 2 oder 3 zur Bekämpfung von Helminthen.

7. Amino-harnstoffderivate der Formel II

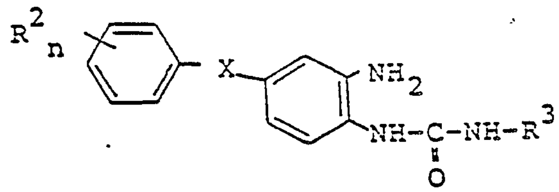

(II)

worin $R^2$, $R^3$, X und n die gleichen Bedeutungen haben wie zu Formel I in Anspruch 1, 2 oder 3.

8. Nitroverbindungen der Formel V

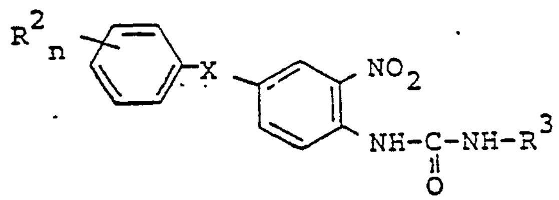

(V)

worin $R^2$, $R^3$, X und n die gleichen Bedeutungen haben wie zu Formel I in Anspruch 1, 2 oder 3.

9. Sulfochloride der Formel VI

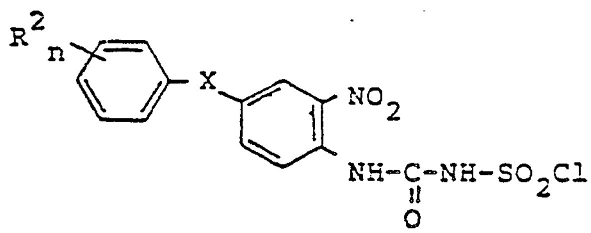

(VI)

worin $R^2$, $R^3$, X und n die gleichen Bedeutungen haben wie zu Formel I in Anspruch 1, 2 oder 3.

10. Verfahren zur Herstellung von substituierten Benzolsulfonsäureestern der Formel I gemäß Anspruch 1, dadurch gekennzeichnet, daß man

a) ein Nitroamin der allgemeinen Formel VII

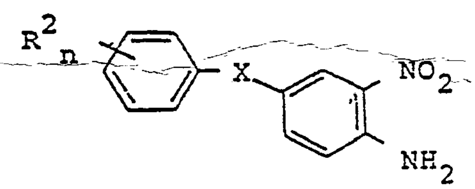

(VII)

mit N-Chlorsulfonylisocyanat $O{=}C{=}N{-}SO_2Cl$ (VIII) umsetzt,

b) das erhaltene Sulfochlorid der Formel VI

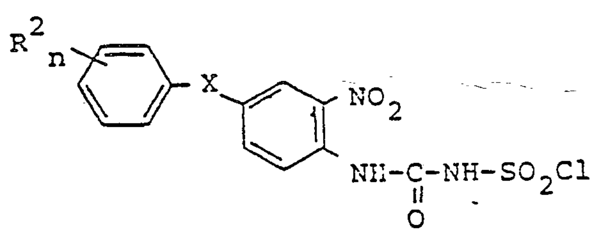

(VI)

hydrolysiert und gegebenenfalls anschließend acyliert,

c) die erhaltene Nitroverbindung der Formel V

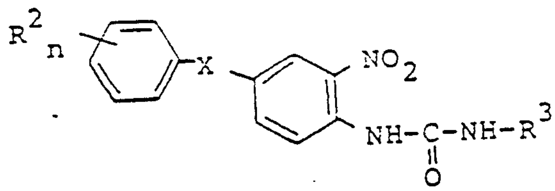

(V)

reduziert und

d) das daraus entstandene Aminoharnstoffderivat der Formel II entweder

i) mit einem Isothiocyanatocarboxylat der Formel S=C=N—COOR[1] (III) zu einer Verbindung der Formel (I) umsetzt, worin Z für =S steht, oder

ii) mit einem Isothioharnstoffcarboxylat der Formel (IV),

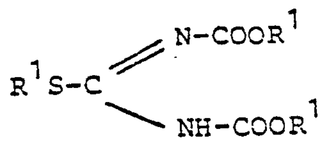

(IV)

in der die über Sauerstoff und über Schwefel gebundenen Reste $R^1$ unabhängig voneinander gleich oder verschieden sein können, in Gegenwart eines Lösungsmittels und einer Säure zu einer Verbindung der Formel I umsetzt, worin Z =N—COOR[1] bedeutet, oder

iii) mit einem Isothioharnstoffcarboxylat der Formel IV zunächst wie nach ii) zu einer Verbindung der Formel I umsetzt, worin Z =N—COOR[1] bedeutet, und diese Verbindung anschließend mit einer Base zu einer Verbindung der Formel I verseift, worin Z für =NH steht, wobei in den Formeln II bis VIII $R^1$ bis $R^4$, X, Z und n die vorgenannten Bedeutungen haben.

11. Verfahren nach Anspruch 4 oder 10, gekennzeichnet durch wenigstens eines der Merkmale

a) daß die Umsetzung der Nitroamine der Formel VII mit dem N-Chlorsulfonylisocyanat VIII zwischen 0 und 20 °C durchgeführt wird,

b) daß die Umsetzung der Nitroamine der Formel VII mit N-Chlorslulfonylisocyanat VIII mit einem 10- bis 20 %igen Überschuß der Verbindung VIII erfolgt,

c) daß die Verseifung der Sulfochloride VI bei 0 bis 80 °C, vorzugsweise in 1 bis 2n HCl erfolgt und

d) daß die Acylierung der Verbindung V, in der $R^3$ Wasserstoff ist, mit einem Überschuß von Säureanhydrid bei 0 bis 40 °C erfolgt, wobei bevorzugt konzentrierte Schwefelsäure als Katalysator verwendet wird.

**Patentanspruch** (für den Vertragsstaat AT)

1. Verfahren zur Herstellung von Benzolsulfonsäureester der Formel I

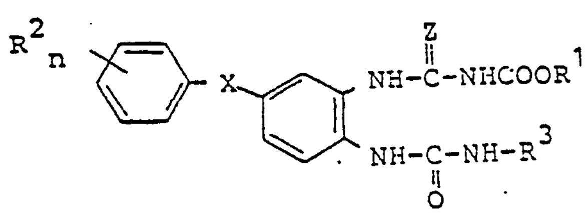

(I)

worin $R^1$ geradkettiges oder verzweiges Alkyl mit 1-4 C-Atomen, n 1, 2 oder 3 und die einzelnen Substituenten $R^2$ unabhängig voneinander Wasserstoff, Fluor, Chlor, Brom, Jod, Cyano, Nitro, Trifluormethyl, 1,1,2,2-Tetrafluorethoxy, geradkettiges oder verzweigtes Alkyl mit 1-12 C-Atomen, Cycloalkyl mit 3-8 C-Atomen, Alkoxy, Alkylthio, Alkylsulfinyl oder Alkylsulfonyl mit jeweils 1-6 C-Atomen im Alkylrest, Acetyl, Acetamino oder jeweils gegebenenfalls ein- oder zweifach halogensubstituiertes Phenyl, Phenoxy, Phenylthio, Phenylsulfinyl oder Phenylsulfonyl bedeuten, $R^3$ Wasserstoff oder —CO—$R^4$ bedeutet, worin $R^4$ gegebenenfalls durch Halogen substituiertes Alkyl oder Alkoxyalkyl mit jeweils 1-6 C-Atomen in einem Alkylteil oder gegebenenfalls durch Halogen, Methyl oder Methoxy substituiertes Phenyl bedeutet, X —$SO_2$—O— oder —O—$SO_2$— und Z =S, N =COOR[1] oder =NH bedeuten, wobei Verbindungen ausgenommen sind, in denen $R^3$ Wasserstoff ist, wenn gleichzeitig Z =N—COOR[1] bedeutet und $R^2$ von 1,1,2,2-Tetrafluoräthoxy, Acetyl, Cycloalkyl und schwefelhaltigen Resten verschieden ist, dadurch gekennzeichnet, daß man

a) ein Nitroamin der allgemeinen Formel VII

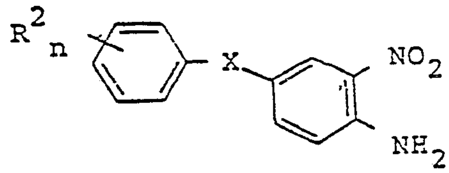

(VII)

mit N-Chlorsulfonylisocyanat $O{=}C{=}N{-}SO_2Cl$ (VIII) umsetzt,

b) das erhaltene Sulfochlorid der Formel VI

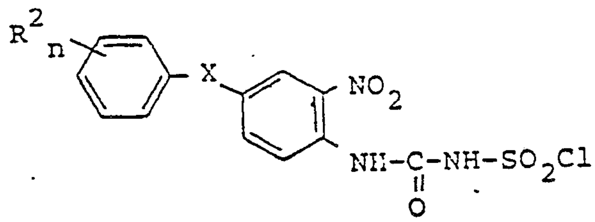

(VI)

hydrolysiert und gegebenenfalls anschließend acyliert,

c) die erhaltene Nitroverbindung der Formel V

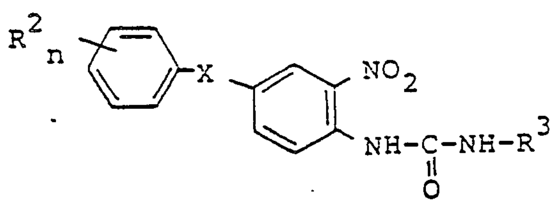

(V)

reduziert und

d) das daraus entstandene Aminoharnstoffderivat der Formel II entweder

i) mit einem Isothiocyanatocarboxylat der Formel $S{=}C{=}N{-}COOR^1$ (III) zu einer Verbindung der Formel (I) umsetzt, worin Z für =S steht, oder

ii) mit einem Isothioharnstoffcarboxylat der Formel (IV),

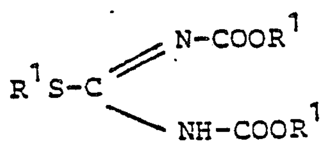

(IV)

in der die über Sauerstoff und über Schwefel gebundenen Reste $R^1$ unabhängig voneinander gleich oder verschieden sein können, in Gegenwart eines Lösungsmittels und einer Säure zu einer Verbindung der Formel I umsetzt, worin $Z = N{-}COOR^1$ bedeutet, oder

iii) mit einem Isothioharnstoffcarboxylat der Formel IV zunächst wie nach ii) zu einer Verbindung der Formel I umsetzt, worin $Z = N{-}COOR^1$ bedeutet, und diese Verbindung anschließend mit einer Base zu einer Verbindung der Formel I verseift, worin Z für =NH steht, wobei in den Formeln II bis VIII $R^1$ bis $R^4$, X, Z und n die vorgenannten Bedeutungen haben.

**Claims** (for the Contracting States : BE, CH, DE, FR, GB, IT, LI, LU, NL, SE)

1. A benzenesulfonic ester of the formula I

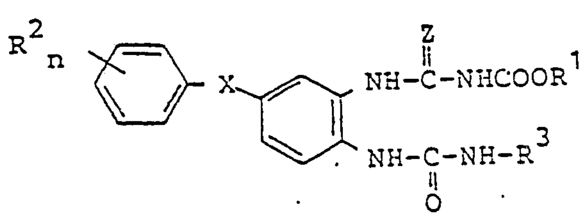

(I)

in which $R^1$ denotes straight-chain or branched alkyl having 1-4 carbon atoms, n denotes 1, 2 or 3 and the individual substituents $R^2$, independently of one another, denote hydrogen, fluorine, chlorine, bromine, iodine, cyano, nitro, trifluoromethyl, 1,1,2,2-tetrafluoroethoxy, straight-chain or branched alkyl having 1-12 carbon atoms, cycloalkyl having 3-8 carbon atoms, alkoxy, alkylthio, alkylsulfinyl or alkylsulfonyl, each having 1-6 carbon atoms in the alkyl radical, acetyl, acetamino, or phenyl, phenoxy, phenylthio, phenylsulfinyl or phenylsulfonyl, each of which is optionally substituted once or twice by halogen, $R^3$ denotes hydrogen or —CO—$R^4$, in which $R^4$ denotes alkyl or alkoxyalkyl which are optionally substituted by halogen, and each of which have 1-6 carbon atoms in an alkyl moiety, or phenyl which is optionally substituted by halogen, methyl or methoxy, X denotes $—SO_2—O—$ or $—O—SO_2—$ and Z denotes =S ; =N—COOR$^1$ or =NH, compounds wherein $R^3$ is hydrogen, when Z simultaneously is =N—COOR$^1$ and $R^2$ is different from 1,1,2,2-tetrafluoroethoxy, acetyl, cycloalkyl and sulfur-containing radicals being excluded.

2. A compound according to formula I in claim 1, in which $R^1$ denotes methyl and $R^2$ denotes hydrogen, fluorine, chlorine, bromine, iodine, trifluoromethyl, 1,1,2,2-tetrafluoroethoxy, straight-chain or branched alkyl having 1-6 carbon atoms or cycloalkyl having 3-6 carbon atoms, and in which $R^3$ denotes hydrogen or —COR$^4$, in which $R^4$ denotes alkyl or alkoxyalkyl, each having 1-4 carbon atoms in an alkyl moiety, or phenyl, X denotes $—SO_2—O—$ or $—O—SO_2—$ and Z denotes =S or $=NCOOCH_3$ and n denotes 1 or 2.

3. A compound according to formula I in claim 1, in which $R^1$ denotes methyl, n denotes 1, $R_2$ denotes 3-trifluoromethyl, $R^3$ denotes hydrogen, X denotes $—SO_2—O—$ and Z denotes $=N—COOCH_3$.

4. A process for the preparation of substituted benzenesulfonic esters of the formula I in claim 1, which comprises reacting an aminourea derivative of the formula II

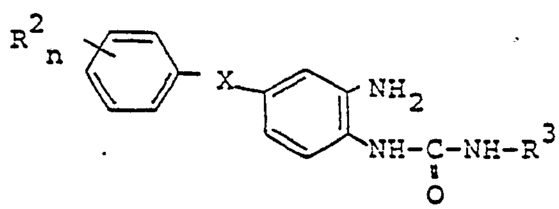

(II)

in which $R^2$, n and $R^3$ have the meanings indicated for formula I in claim 1, either

a) with an isothiocyanatocarboxylate of the formula III, S=C=N—COOR$^1$ III in which $R^1$ has the abovementioned meaning, to give a compound of the formula I, in which Z represents =S,

b) with an isothioureidocarboxylate of the formula IV

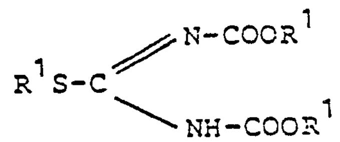

(IV)

in which $R^1$ has the abovementioned meaning, and the radicals $R^1$ which are bonded via oxygen and via sulfur can, independently of one another, be identical or different, in the presence of a solvent and an acid to give a compound of the formula I, in which Z denotes =N—COOR$^1$,

c) with an isothioureidocarboxylate of the formula IV, initially as in b) to give a compound of the formula I in which Z denotes =N—COOR$^1$, and then hydrolyzing this compound with a base to give a compound of the formula I in which Z represents =NH.

5. Pharmaceutical preparation containing as the active ingredient a substituted benzenesulfonic ester of the formula I in claim 1, 2 or 3 in admixture or conjunction with a customary pharmaceutical vehicle or mixed with a customary feedstuff.

6. Method of combating helminths by administering to the patient a sufficient amount of a substituted benzenesulfinic ester of the formula I in claim 1, 2 or 3.

7. Aminourea derivatives of the formula II

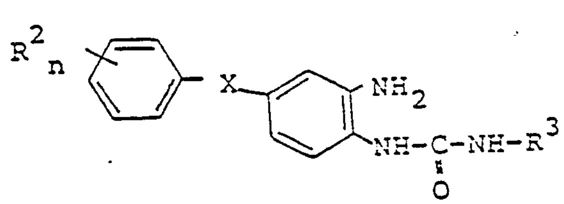

(II)

in which $R^2$, $R^3$, X and n have the same meanings as for formula I in claim 1, 2 or 3.

8. Nitrocompounds of the formula V

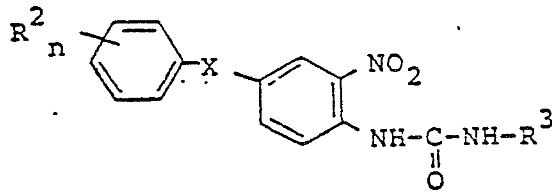

(V)

in which $R^2$, $R^3$, X and n have the same meanings as for formula I in claim 1, 2 or 3.

9. Sulfochlorides of the formula VI

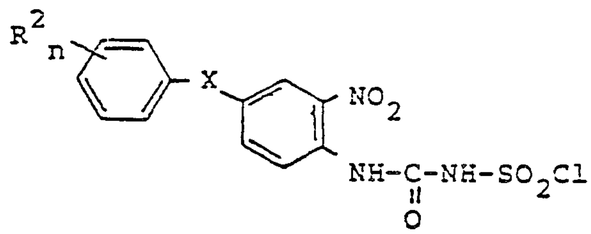

(VI)

in which $R^2$, $R^3$, X and n have the same meanings as for formula I in claim 1, 2 or 3.

10. Process for the preparation of substituted benzenesulfonic esters of the formula I according to claim 1, which comprises

a) reacting a nitroamine of the general formula VII

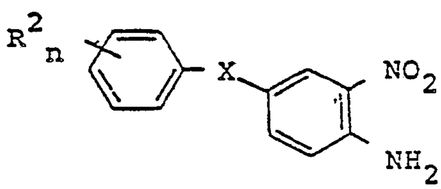

(VII)

with N-chlorosulfonylisocyanate $O=C=N-SO_2Cl$ (VIII),

b) hydrolyzing the resulting sulfochloride of the formula VI

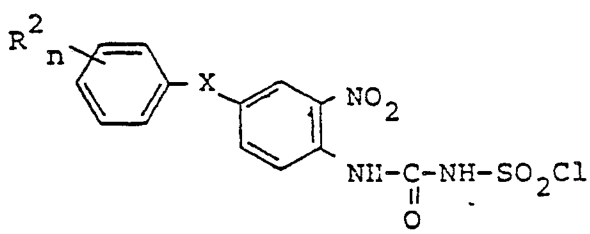

(VI)

optionally followed by acylation,

c) reducing the resulting nitro compound of the formula V

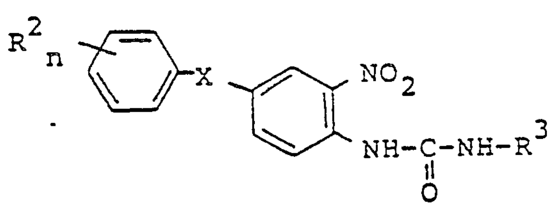

(V)

and

d) reacting the aminourea derivative obtained therefrom of the formula II either

i) with an isothiocyanatocarboxylate of the formula $S=C=N-COOR^1$ (III) to give a compound of the formula I, wherein Z is =S or

ii) with an isothioureacarboxylate of the formula IV

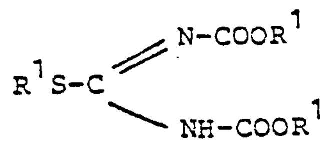

(IV)

wherein the radicals $R^1$ bound via an oxygen or sulfur atom may be the same or different from one another, independently from each other, in the presence of a solvent and an acid to give a compound of the formula I, wherein Z is =N—COOR$^1$, or

iii) with an isothioureacarboxylate of the formula IV in accordance with ii) to give a compound of the formula I, wherein Z is =N—COOR$^1$ and subsequently saponifying this compound with a base to give a compound of the formula I, wherein Z is =NH, the radicals $R^1$ to $R^4$, X, Z and n having the aforesaid meanings.

11. The process according to claim 4 or 10, characterized by at least one of the following features

a) reaction of the nitroamines of the formula VII with N-chlorosulfonylisocyanate VIII at a temperature between 0 and 20 °C,

b) reaction of the nitroamines of the formula VII with N-chlorosulfonylisocyanate VIII with a 10 — to 20 % excess of the compound VIII,

c) saponification of the sulfochlorides VI at a temperature of from 0 to 80 °C, preferably in 1 to 2 N HCl and

d) acylation of the compound V, wherein $R^3$ is hydrogen, with excess acid anhydride at 0 to 40 °C, concentrated sulfuric acid being used preferably as a catalyst.

**Claim** (for the Contracting State AT)

1. A process for the preparation of benzenesulfonic esters of the formula I

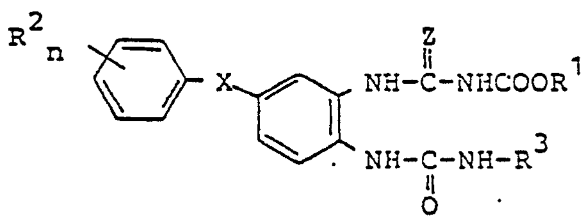

(I)

in which $R^1$ denotes straight-chain or branched alkyl having 1-4 carbon atoms, n denotes 1, 2 or 3 and the individual substituents $R^2$, independently of one another, denote hydrogen, fluorine, chlorine, bromine, iodine, cyano, nitro, trifluoromethyl, 1,1,2,2-tetrafluoroethoxy, straight-chain or branched alkyl having 1-12 carbon atoms, cycloalkyl having 3-8 carbon atoms, alkoxy, alkylthio, alkylsulfinyl or alkylsulfonyl, each having 1-6 carbon atoms in the alkyl radical, acetyl, acetamino, or phenyl, phenoxy, phenylthio, phenylsulfinyl or phenylsulfonyl, each of which is optionally substituted once or twice by halogen, $R^3$ denotes hydrogen or —CO—$R^4$, in which $R^4$ denotes alkyl or alkoxyalkyl which are optionally substituted by halogen, and each of which have 1-6 carbon atoms in an alkyl moiety, or phenyl which is optionally substituted by halogen, methyl or methoxy, X denotes —$SO_2$—O— or —O—$SO_2$— and Z denotes =S ; =N—COOR$^1$ or =NH, compounds wherein $R^3$ is hydrogen, when Z simultaneously is =N—COOR$^1$ and $R^2$ is different from 1,1,2,2-Tetrafluoroethoxy, acetyl, cycloalkyl and sulfur-containing radicals being excluded, which comprises

a) reacting a nitroamine of the general formula VII

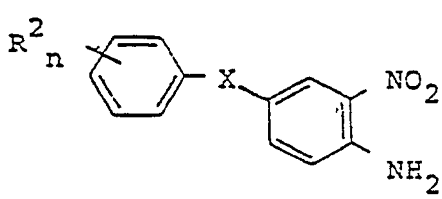

(VII)

with N-chlorosulfonylisocyanate O=C=N—$SO_2$Cl (VIII),

b) hydrolyzing the resulting sulfochloride of the formula VI

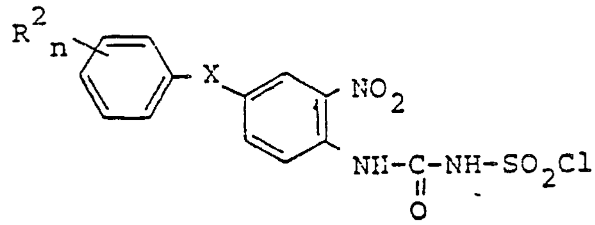

(VI)

optionally followed by acylation,

c) reducing the resulting nitro compound of the formula V

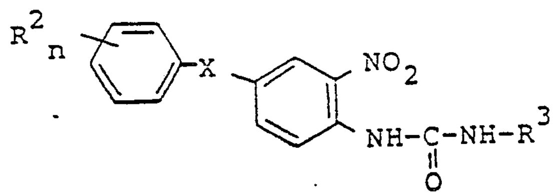

(V)

and

d) reacting the aminourea derivative obtained therefrom of the formula II either

i) with an isothiocyanatocarboxylate of the formula S=C=N—COOR[1] (III) to give a compound of the formula I, wherein Z is =S or

ii) with an isothioureacarboxylate of the formula IV

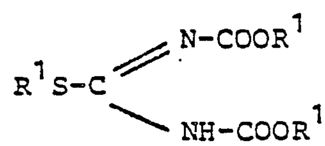

(IV)

wherein the radicals $R^1$ bound via an oxygen or sulfur atom may be the same or different from one another, independently from each other, in the presence of a solvent and an acid to give a compound of the formula I, wherein Z is =N—COOR[1], or

iii) with an isothioureacarboxylate of the formula IV in accordance with ii) to give a compound of the formula I, wherein Z is =N—COOR[1] and subsequently saponifying this compound with a base to give a compound of the formula I, wherein Z is =NH, the radicals $R^1$ to $R^4$, X, Z and n having the aforesaid meanings.

**Revendications** (pour les Etats contractants : BE, CH, DE, FR, GB, IT, LI, LU, NL, SE)

1. Esters d'acide benzène sulfonique substitué de formule I

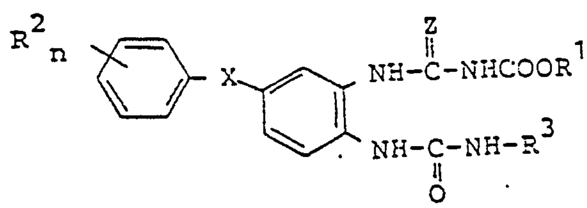

(I)

dans laquelle $R^1$ représente un groupe alcoyle à chaîne droite ou ramifiée ayant de 1 à 4 atomes de carbone, n est 1, 2 ou 3 et les substituants individuels $R^2$ représentent indépendamment les uns des autres l'hydrogène, le fluor, le chlore, le brome, l'iode, un groupe cyano, nitro, trifluorométhyle, 1,1,2,2-tétrafluoroéthoxy, un groupe alcoyle à chaîne droite ou ramifiée ayant de 1 à 12 atomes de carbone, un groupe cycloalcoyle ayant de 3 à 8 atomes de carbone, un groupe alcoxy, alcoylthio, alcoylsulfinyle ou alcoyl-sulfonyle ayant chacun de 1 à 6 atomes de carbone dans la partie alcoyle, un groupe acétyle, acétamino ou un groupe phényle, phénoxy, phénylthio, phénylsulfinyle ou phénylsulfonyle éventuellement 1 ou 2 fois substitué par un halogène, $R^3$ représente l'hydrogène ou —CO—$R^4$, où $R^4$ représente un groupe alcoyle ou alcoxyalcoyle éventuellement substitué par un halogène, ayant de 1 à 6 atomes de

carbone dans la partie alcoyle, ou un groupe phényle éventuellement substitué par un halogène, un groupe méthyle ou méthoxy, X représente $—SO_2—O—$ ou $—O—SO_2—$ et Z est =S, $=N—COOR^1$ ou =NH, des composés dans lesquels $R^3$ représente l'hydrogène, lorsque Z représente simultanément $=N—COOR^1$ et $R^2$ est autre que les groupes 1,1,2,2-tétrafluoroéthoxy, acétyle, cycloalcoyle et des radicaux soufrés étant exclus.

2. Composés de formule I suivant la revendication 1 caractérisés en ce que $R^1$ représente un groupe méthyle et $R^2$ représente l'hydrogène, le fluor, le chlore, le brome, l'iode, un groupe trifluorométhyle, 1,1,2,2-tétrafluoroéthoxy, un groupe alcoyle à chaîne droite ou ramifiée ayant de 1 à 6 atomes de carbone ou un groupe cycloalcoyle ayant de 3 à 6 atomes de carbone et $R^3$ représente l'hydrogène ou $—COR^4$ où $R^4$ est un groupe alcoyle ou alcoxyalcoyle ayant de 1 à 4 atomes de carbone dans la partie alcoyle, ou un groupe phényle, X représente $—SO_2—O$ ou $—O—SO_2—$ et Z représente =S ou $=NCOOCH_3$ et n est 1 ou 2.

3. Composés de formule I suivant la revendication 1, caractérisés en ce que $R^1$ représente un groupe méthyle, n est 1, $R^2$ représente un groupe 3-trifluorométhyle, $R^3$ représente l'hydrogène, X représente le groupe $—SO_2—O—$ et Z représente un groupe $=N—COOCH_3$.

4. Procédé de préparation d'esters d'acide benzène sulfonique substitué de formule I suivant la revendication 1, caractérisé en ce qu'on fait réagir un dérivé d'aminourée de formule II

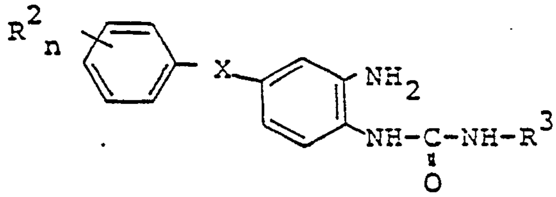

dans laquelle $R^2$, n et $R^3$ ont les significations indiquées pour la formule I suivant la revendication 1 soit

a) avec un isothiocyanatocarboxylate de formule III $S=C=N—COOR^1$ III dans laquelle $R^1$ a la signification indiquée ci-dessus, pour obtenir un composé de formule I dans lequel Z est =S, soit

b) avec un isothiouréecarboxylate de formule IV

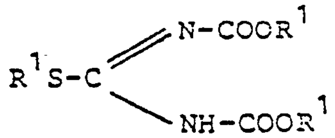

(IV)

dans laquelle $R^1$ a la signification indiquée ci-dessus et les radicaux $R^1$ fixés par l'intermédiaire du soufre et de l'oxygène peuvent être indépendamment les uns des autres identiques ou différents, en présence d'un solvant et d'un acide, pour obtenir un composé de formule I dans lequel Z est $=N—COOR^1$, soit

c) avec un isothiourée carboxylate de formule IV, d'abord comme en b) pour obtenir un composé de formule I, dans lequel z est $=N—COOR^1$, puis on saponifie ce composé avec une base en un composé de formule I dans lequel Z est =NH.

5. Composition pour combattre des helminthes, caractérisée en ce qu'elle renferme un ester d'acide benzène sulfonique substitué de formule I suivant l'une quelconque des revendications 1, 2 ou 3, en mélange avec un véhicule pharmaceutique usuel ou en mélange avec un aliment pour bétail usuel.

6. Utilisation d'un ester d'acide benzène sulfonique de formule I suivant l'une quelconque des revendications 1, 2 ou 3 pour combattre des helminthes.

7. Des dérivés d'aminourée de formule II

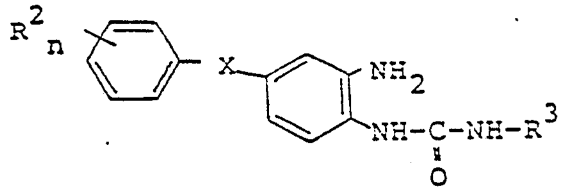

dans laquelle $R^2$, $R^3$, X et n ont les mêmes significations que dans la formule I suivant l'une quelconque des revendications 1, 2 ou 3.

8. Des composés nitro de formule V

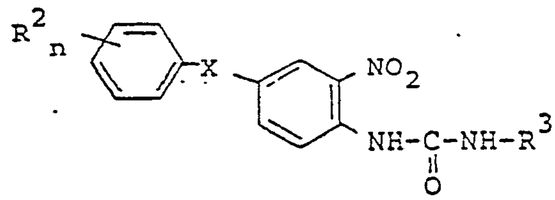

dans laquelle $R^2$, $R^3$, X et n ont les mêmes significations que dans la formule I suivant l'une quelconque des revendications 1, 2 ou 3.

9. Sulfochlorures de formule VI

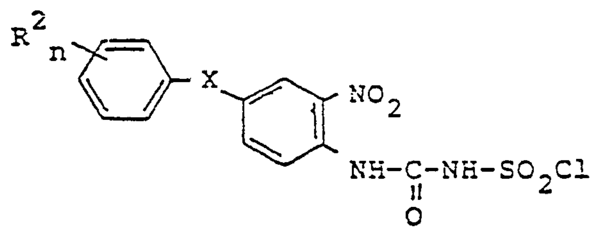

dans laquelle $R^2$, $R^3$, X et n ont les mêmes significations que dans la formule I suivant l'une quelconque des revendications 1, 2 ou 3.

10. Procédé de préparation d'esters d'acide benzène sulfonique substitué de formule I suivant la revendication 1, caractérisé en ce

a) qu'on fait réagir une nitroamine de formule générale VII

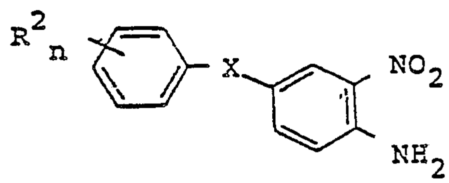

avec un isocyanate de N-chlorosulfonyle $O{=}C{=}N{-}SO_2Cl$ (VIII),

b) qu'on hydrolyse le sulfochlorure formé de formule VI

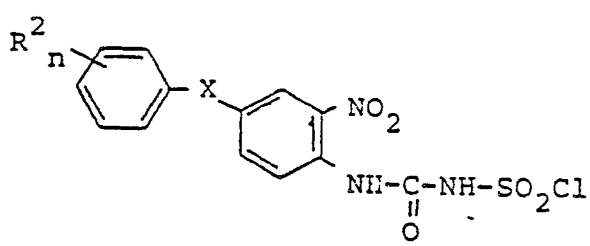

le cas échéant suivi d'une acylation,

c) qu'on réduit le composé nitro de formule V formé

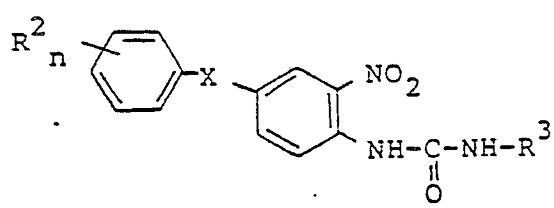

et

d) qu'on fait réagir le dérivé d'aminourée formé de formule II soit

i) avec un isothiocyanato carboxylate de formule $S{=}C{=}N{-}COOR^1$ (III) pour obtenir un composé de formule (I), dans lequel Z représente =S, soit

ii) avec un isothiourée carboxylate de formule (IV)

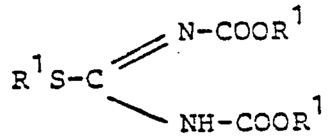

dans lequel les radicaux $R^2$ liés par l'oxygène ou le soufre peuvent être indépendamment les uns des autres identiques ou différents, en présence d'un solvant et d'un acide pour obtenir un composé de formule I, dans lequel Z représente $=N-COOR^1$, soit

iii) avec un isothiourée carboxylate de formule IV d'abord selon ii) pour obtenir un composé de formule I, dans lequel Z représente $=N-COOR^1$, puis on saponifie ce composé avec une base pour obtenir un composé de formule I, dans lequel Z représente =NH, les radicaux $R^1$ à $R^4$, X, Z et n dans les formules II à VIII ayant les significations indiquées ci-dessus.

11. Procédé suivant l'une des revendications 4 et 10, caractérisé par au mois l'une des caractéristiques suivantes :

a) réalisation de la réaction des nitroamines de formules VII avec l'isocyanate de N-chlorosulfonyle VIII à une température entre 0 et 20 °C,

b) réalisation de la réaction des nitroamines de formule VII avec l'isocyanate de N-chlorosulfonyle VIII avec un excès de 10 à 20 %, du composé VIII,

c) réalisation de la saponification des sulfochlorures VI à une température de 0 à 80 °C, de préférence dans HCl 1 à 2 N et

d) réalisation de l'acylation du composé V, dans lequel $R^3$ représente l'hydrogène, avec un excès de l'anhydride d'acide, à 0 à 40 °C, en utilisant de préférence l'acide sulfurique concentré comme catalyseur.

**Revendication** (pour l'Etat contractant AT)

1. Procédé de préparation d'esters d'acide benzène sulfonique substitué de formule I

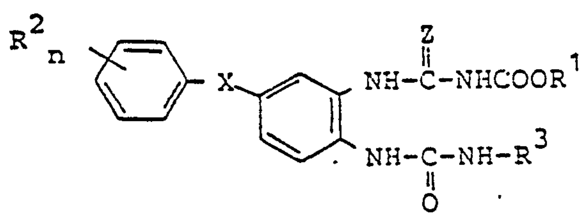

(I)

dans laquelle $R^1$ représente un groupe alcoyle à chaîne droite ou ramifiée ayant de 1 à 4 atomes de carbone, n est 1, 2 ou 3 et les substituants individuels $R^2$ représentent indépendamment les uns des autres l'hydrogène, le fluor, le chlore, le brome, l'iode, un groupe cyano, nitro, trifluorométhyle, 1,1,2,2-tétrafluoroéthoxy, un groupe alcoyle à chaîne droite ou ramifiée ayant de 1 à 12 atomes de carbone, un groupe cycloalcoyle ayant de 3 à 8 atomes de carbone, un groupe alcoxy, alcoylthio, alcoylsulfinyle ou alcoyl-sulfonyle ayant chacun de 1 à 6 atomes de carbone dans la partie alcoyle, un groupe acétyle, acétamino ou un groupe phényle, phénoxy, phénylthio, phénylsulfinyle ou phénylsulfonyle éventuellement 1 ou 2 fois substitué par un halogène, $R^3$ représente l'hydrogène ou $-CO-R^4$, où $R^4$ représente un groupe alcoyle ou alcoxyalcoyle éventuellement substitué par un halogène, ayant de 1 à 6 atomes de carbone dans la partie alcoyle, ou un groupe phényle éventuellement substitué par un halogène, un groupe méthyle ou méthoxy, X représente $-SO_2-O-$ ou $-O-SO_2-$ et Z est =S, $=N-COOR^1$ ou =NH, des composés dans lesquels $R^3$ représente l'hydrogène, lorsque Z représente simultanément $=N-COOR^1$ et $R^2$ est autre que des groupes 1,1,2,2-tétrafluoroéthoxy, acétyle, cycloalcoyle et des radicaux soufrés étant exclus, caractérisé en ce

a) qu'on fait réagir une nitroamine de formule générale VII

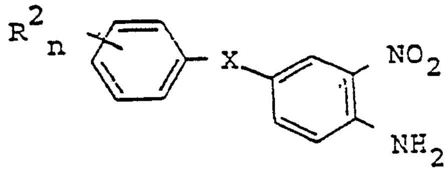

avec un isocyanate de N-chlorosulfonyle $O=C=N—SO_2Cl$ (VIII),

b) qu'on hydrolyse le sulfochlorure formé de formule VI

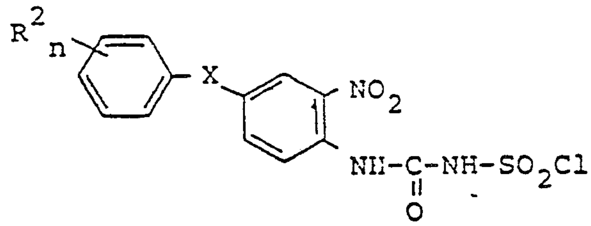

le cas échéant suivi d'une acylation,

c) qu'on réduit le composé nitro de formule V formé

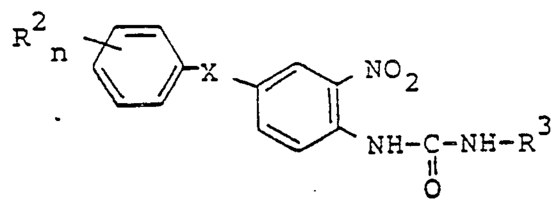

et

d) qu'on fait réagir le dérivé d'aminourée formé de formule II soit

i) avec un isothiocyanato carboxylate de formule $S=C=N—COOR^1$ (III) pour obtenir un composé de formule (I), dans lequel Z représente =S, soit

ii) avec un isothiourée carboxylate de formule (IV)

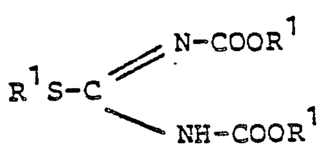

dans lequel les radicaux $R^2$ liés par l'oxygène ou le soufre peuvent être indépendamment les uns des autres identiques ou différents, en présence d'un solvant et d'un acide pour obtenir un composé de formule I, dans lequel Z représente $=N—COOR^1$, soit

iii) avec un isothiourée carboxylate de formule IV d'abord selon ii) pour obtenir un composé de formule I, dans lequel Z représente $=N—COOR^1$, puis on saponifie ce composé avec une base pour obtenir un composé de formule I, dans lequel Z représente =NH, les radicaux $R^1$ à $R^4$, X, Z et n dans les formules II à VIII ayant les significations indiquées ci-dessus.